# EUROPEAN PATENT APPLICATION

(11) **EP 2 827 123 A1**
(43) Date of publication of application: **21.01.2015**
(21) Application number: 14176876.2
(22) Date of filing: 14.07.2014
(51) Int. Cl.: G01N 1/20, B07B 13/11, G01N 3/32, G01N 33/00

(54) **Accelerated powder segregation testing apparatus and method**

(30) Priority: 17.07.2013 US 201361847102 P
(71) Applicant: Kalidindi, Sanyasi R., Monroe, NJ 08831 (US)
(72) Inventor: Kalidindi, Sanyasi R., Monroe, NJ 08831 (US)
(74) Representative: Grey, Ian Michael

(57) **Abstract**

An accelerated powder segregation testing apparatus and method are disclosed. The apparatus consists of an angularly oriented ramp 10 mounted on a vibration device 22. The powder to be tested is fed into an origin portion 14 of the ramp 10, and when the vibrator 22 is activated, the powder moves along the ramp 10. As the powder exits after the test, unit-dose powder samples are collected, compacted and analyzed for differential in content uniformity. By comparing the results from several formulations, the formulation that is most resistant to segregation can be selected for production.

## Description

### RELATED APPLICATION:

This application is a conversion of and claims priority from provisional patent application No. 61/847,102 filed July 17, 2013.

### FIELD OF THE INVENTION:

The present invention relates to the field of laboratory apparatus and methods for testing the segregation of powder blends, and more particularly for accelerated testing of powder blends for component segregation.

### BACKGROUND OF THE INVENTION:

Many pharmaceutical, food, cosmetic and other chemical formulations are made by mixing component powders or granules to form a powder blend prior to further processing, e.g. compression into tablets, filling capsules, bottles or pouches. While the blended powder initially is formed at an acceptable level of content uniformity, further handling, which may involve auger feeding, vibration feeding, screw feeding, vacuum transfer, etc., often results in segregation of the ingredients, ultimately leading to unacceptable content uniformity from one package to the next. As an example, if one of the ingredients in a powder drink mix is a sweetener, drinks mixed from different containers packaged after segregation caused by handling are likely to have taste differentiation. Similarly, in a pharmaceutical powder blend at least one ingredient is an active ingredient, i.e. a drug, where segregation is likely to render the blend non-uniform, and individual doses to be potentially ineffective or dangerous. Standards used in the pharmaceutical industry require not more than a 6.0% variation from a stated quantity of active ingredient. Powder blends should be tested for segregation in the laboratory during the stage of formulation development. Once the product is in production scale manufacturing, a segregation problem would be more difficult to solve and correct because of regulatory and economic constraints. However, at the formulation development stage, batch sizes are typically small and processing time of such small batches is typically too short for a segregation problem to be detected by presently known apparatus and methods. Thus, an accelerated testing apparatus and method, adapted to induce segregation of blended powders relatively quickly, would be very useful to the formulators in order to enable detection and correction of potential segregation problems early in the process.

An Apparatus And Method For Testing Powder Properties is disclosed in U.S. Patent No. 5,583,304 to the present inventor. The apparatus disclosed is mounted within a three compartment housing that has a hopper connected to a programmable vibrator to simulate production conditions. The hopper has a rotatable butterfly valve in the exit chute. A carousel with multiple stations for receiving samples is mounted below the hopper exit chute and caused to rotate cyclically. There are fundamental problems with this apparatus which render it substantially ineffective, for example:
(a) studying segregation properties of a static powder bed, such as the one provided by this apparatus, does not provide meaningful results because the powder tends to pack;
(b) the flow of powder from the hopper is impeded because the powder path becomes narrower in width from the stem of the hopper to the stem of the funnel die;
(c) the flow of powder is also hampered by the butterfly valve in the stem of the hopper; and
(d) reproducibility of vibration intensity is unreliable because the vibration device is mounted to the wall of an enclosure that is remote from the hopper.

Another device of the present inventor, Apparatus For Testing Powder Properties, is disclosed in U.S. Patent No. 7,204,164. This apparatus provides an improvement on the apparatus of U.S. Patent No. 5,583,304 by:
(a) improving the powder flow by altering the geometry of the stem of the hopper, and incorporating a new gate system for controlling the powder flow;
(b) providing an improved technique for taking multiple unit-dose samples of the powder at predetermined intervals during testing;
(c) directly linking the vibrator to the hopper to cause vibration to be transmitted directly to the hopper, causing the vibration intensity to be reproducible; and
(d) providing a novel apparatus for studying segregation potential, and also for testing particle size distribution and flow rates of powders, adding versatility to the apparatus.

This apparatus, though much improved in design than the apparatus of U.S. Patent No. 5,583,304, suffers from the following drawbacks:
(a) a large sample size (1-3 kg) is required for meaningful testing;
(b) flow of the powder blend from the hopper stem is inconsistent;
(c) different formulations of the same product may require different gate openings for acceptable flow, potentially biasing the results; and
(d) the apparatus is cumbersome.

Therefore, a need exists for a simple apparatus and method to perform accelerated segregation testing of powder blends.

### SUMMARY OF THE INVENTION:

A ramp is provided in the form of a helix that is supported on a column. The helical ramp is connected to an agitator, e.g. a vibrator, which can be controlled for frequency and amplitude. A quantity of powder blend is prepared containing an active ingredient, a filler/binder, a disintegrant and a lubricant. A portion of the powder blend is placed at the origin of the ramp and the agitator is activated, causing the powder to travel along the ramp, depending on the direction of travel induced by the vibration, and partially segregate the powder blend into components. The powder is collected at the output end of the ramp in unit doses. Each unit dose is tested for residual components, and a determination of degree of segregation, i.e. uniformity, is made. Testing of various powder blends is conducted to achieve an acceptable blend for production.

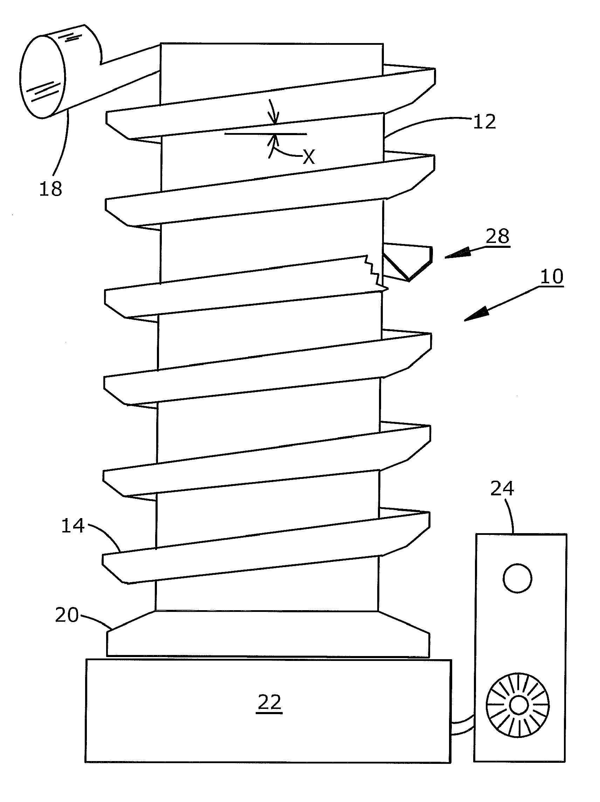

### BRIEF DESCRIPTION OF THE DRAWING:

The present invention is best understood in conjunction with the accompanying drawing figure showing a front elevation view of the apparatus of the invention for testing a powder blend for segregation, the apparatus has a helix supported by a central column.

### DETAILED DESCRIPTION OF THE PREFERRED EMBODIMENT:

Referring to the drawing figure, a ramp 10 is supported by a column 12. Column 12 is mounted on a base 20, which, in turn is mounted on an agitator 22. Ramp 10 travels from an origin 14 at a lower end to an output chute 18 at an upper end. In an alternate embodiment of the invention, the origin is located at the upper end of the ramp and the output is located at the lower end; the difference depending on the apparatus being adapted for upward or downward travel. In order to provide an effectively long ramp 10 in a relatively small amount of surface area, ramp 10 is in the form of a helix. Alternate forms of ramp, e.g. a linear ramp, are similarly effective for the method described below. In helical form, ramp 10 is fixedly attached circumferentially around column 12, e.g. by welding. The upward angle X of ramp 10 is substantially uniform throughout its length and at a preferred angle of between approximately 5.0° and 10.0°, and most preferably approximately 7.5°. As shown in a cutaway section seen at number 28, ramp 10 is formed as a channel having a "V" shaped contour. Alternate contour shapes, e.g. curved, are understood to be within the scope of the present invention. Agitator 22 is preferably a vibrator having a control 24 connected thereto in order to vary oscillation amplitude and/or frequency. Whereas alternate modes of agitation are possible, it has been determined that vibration tends to be most repeatable, particularly with the vibrator directly mounted to the object apparatus.

Operation of the apparatus of the present invention begins by placing a quantity of blended powder on ramp 10 at origin 14 for segregation testing. As noted above, the origin may be at the top with powder travel downward. Control 24 is activated to cause agitator 22 to vibrate column 12 and ramp 10. Agitator 22 is able to generate vibratory pulses, the pulses preferably being rotational in character. With ramp 10 and column 12 being vibrated by agitator 22, the charge of approximately 10g of blended powder is placed on ramp 10 at origin 14 to travel counterclockwise (as viewed from above) moving upward along ramp 10. As discussed briefly above, the powder blend will become partially segregated under the applied agitation, and the powder will partially separate into the original components. The major objective of the segregation test is to determine the uniformity of active ingredient remaining in the blend in order to predict the efficacy of the ultimate packaged form.

Typical formulations of a specific pharmaceutical to be tested for segregation of the powder blend are presented in the table below.

| COMPONENT TYPE | QUANTITY | BLEND A | BLEND B | BLEND C |
|---|---|---|---|---|
| Active | 80.0 g | Acetominaphen micronized | Acetominaphen micronized | Acetominaphen micronized |
| Filler/Binder 1 | 932.0 g | Microcrystalline cellulose | Microcrystalline cellulose | PanExcea® * |
| Filler/Binder 2 | 928.0 g | Dicalcium phosphate dehydrate | Lactose monohydrate spray-dried | PanExcea® * |
| Disintegrant | 40.0 g | Croscarmellose sodium, NF | Croscarmellose sodium, NF | Croscarmellose sodium, NF |
| Lubricant | 20.0 g | Magnesium stearate, NF | Magnesium stearate, NF | Magnesium stearate, NF |
| Total Weight | 2,000.0 g | | | |

| | | | | |
|---|---|---|---|---|
| *PanExcea® is a co-processed filler containing microcrystalline cellulose, corspovidone and hydroxypropyl methylcellulose. | | | | |

Each of the above Blends A, B, C were subjected to processing in the apparatus described above. The vibration agitator was set at a frequency of 47 Hz and the amplitude was varied. Subsequent to subjecting each powder blend to agitation and the resultant segregation, samples were obtained in package sizes or unit doses. A typical unit dose process is prepared by capturing small quantities of the segregated powder blend in a tablet press device, such as is described in U.S. Patent No. 6,585,507. The tablets are tested for content uniformity in accordance with the desired formulation, e.g. by spectrographic analysis. The relative standard deviation (RSD) results are displayed in the following chart.

| % Amplitude | 50% | 55% | 62% |
|---|---|---|---|
| Blend A | 9.67 RSD | 5.48 RSD | 5.16 RSD |
| Blend B | 4.16 RSD | 2.46 RSD | 3.03 RSD |
| Blend C | 2.36 RSD | 3.24 RSD | 1.34 RSD |

The greater the RSD, the greater the degree of segregation of the active ingredient. In the test results above, Blend A shows a greater RSD than either Blend B or Blend C at each of the amplitude percent variations indicated. This result correlates with the filler/binder being a combination of microcrystalline cellulose and dicalcium phosphate dehydrate, due to these ingredients differing significantly from one another in particle size and in bulk density. Blend C shows the lowest RSD, likely a result of the PanExcea@ component that is a co-processed mixture having a high degree of particle uniformity. It will be noticed that the degree of segregation, expressed as RSD, is not linear in respect to the percent amplitude setting. Therefore, the formulator must establish a standard for the most effective and repeatable test result for a given powder blend.

The objective of the present invention is to provide a testing apparatus and method for evaluating the relative segregation of blended powders to be utilized in establishing manufacturing criteria. The apparatus and method provided function effectively at a relatively small sample batch size, allowing laboratory determination of powder blend segregation at an accelerated rate. By determining a powder blend that is relatively stable, i.e. low degree of segregation, the ultimate product, either pharmaceutical, food, cosmetic, or other, can be made with a high degree of uniformity from package to package.

While the description above discloses a preferred embodiment of the present invention, it is contemplated that numerous variations of the invention are possible and are considered to be within the scope of the claims that follow.

## Claims

1. An apparatus for accelerated powder segregation testing, comprising:
a. a ramp 10 oriented at an angle X, the ramp 10 having an origin 14 and an output 18; and
b. an agitator 22 connected to the ramp 10 in a manner to cause the ramp 10 to vibrate when the agitator 22 is activated.

2. The apparatus described in claim 1, wherein the ramp 10 is in the form of a helix and the ramp 10 is supported on a column 12.

3. The apparatus described in claim 1, wherein the ramp 10 is formed with a "V" shape 28 in cross section.

4. The apparatus described in claim 1, further comprising a control 24 connected to the agitator 22.

5. The apparatus described in claim 1, wherein the output 18 is higher than the origin 14.

6. The apparatus described in claim 1, wherein angle X is between approximately 5.0° and 10.0°.

7. The apparatus described in claim 6, wherein angle X is approximately 7.5°.

8. The apparatus described in claim 1, wherein the agitator 22 is capable of being varied by the control 24 for amplitude and frequency.

9. A method for accelerated segregation testing of powder blends, comprising the steps of:
a. providing a testing apparatus having an angled ramp 10 and an agitator 22, the ramp 10 having an origin 14 and an output 18;
b. placing a quantity of a powder blend on the ramp 10 at the origin 14;
c. activating the agitator 22 to vibrate the ramp 10;
d. receiving the quantity of powder blend at the output 18; and
e. evaluating the powder blend after segregation for uniformity.

10. The method described in claim 9, further comprising controlling the agitator 22 for amplitude and frequency of oscillation.

11. The method described in claim 9, further comprising the step of repeating the process at various levels of oscillation amplitude and frequency to determine a powder blend with minimum degree of ingredient segregation.
